# EUROPÄISCHE PATENTSCHRIFT

(11) **EP 1 500 649 B1**
(45) Veröffentlichungstag und Bekanntmachung des Hinweises auf die Patenterteilung: **19.06.2013**
(21) Anmeldenummer: 04016925.2
(22) Anmeldetag: 18.07.2004
(51) Int. Cl.: C07D 213/30, C07C 1/26, C07C 41/30, C07F 7/08

(54) **Verfahren zur Durchführung einer In-Situ-Quench Reaktion**
Process for performing an in-situ-quench reaction
Procédé pour la mise en oeuvre d'une réaction d'arrêt in-situ

(30) Priorität: 22.07.2003 DE 10333174
(43) Veröffentlichungstag der Anmeldung: 26.01.2005
(73) Patentinhaber: Lonza AG, 4052 Basel (CH)
(72) Erfinder: Schmalz, Hans-Günther, Prof. Dr., 50321 Brühl (DE); Schwalbe, Thomas, Dr., 61118 Bad Vilbel (DE); Sakamoto, Yuki, Hyogo 661-0026 (JP); Matsumoto, Keisuke, Fukui City, Fukui 910-0066 (JP); Goto, Sachio, Ikoma-City, Nara 630-0254 (JP)
(74) Vertreter: Kompter, Hans-Michael

(56) Entgegenhaltungen:
- EP-A- 1 160 241
- WO-A-02/068403
- W. LI ET AL.: "An improved protocol for the preparation of 3-pyridyl- and some arylboronic acids." JOURNAL OF ORGANIC CHEMISTRY., Bd. 67, 2002, Seiten 5394-5397, XP002300451 AMERICAN CHEMICAL SOCIETY. EASTON.
- SUZUKI H ET AL: "ENANTIOSELECTIVE SYNTHESIS OF (R)- AND (S)-4-BENZYLOXY-2-CYCLOHEXEN-1-ON" JOURNAL OF ORGANIC CHEMISTRY, AMERICAN CHEMICAL SOCIETY. EASTON, US, Bd. 66, Nr. 4, 23. Februar 2001 (2001-02-23), Seiten 1494-1496, XP001001780 ISSN: 0022-3263
- E.J. COREY ET AL.: "Highly selective, kinetically controlled enolate formation using lithium dialkylamides in the presence of trimethylchlorosilane." TETRAHEDRON LETTERS, ELSEVIER SCIENCE PUBLISHERS, AMSTERDAM, NL, Bd. 25, Nr. 5, 1984, Seiten 495-498, XP002300452 ISSN: 0040-4039
- W.A. HERRMANN ET AL.: "Chiral molybdenum(VI) and tungsten(VI) 2'-pyridinyl alcoholate complexes. Synthesis, structure and catalytic properties in asymmetric olefin epoxidation." JOURNAL OF ORGANOMETALLIC CHEMISTRY., Bd. 603, Nr. 1, 2000, Seiten 69-79, XP002300453 CHELSEVIER-SEQUOIA S.A. LAUSANNE.
- [Online] Seiten 1 - 4 Gefunden im Internet: <URL:http://de.wikipedia.org/wiki/lithiumor ganyl>
- U. Wietelmann: "Lithium and lithium compounds" In: "Ullmann's Encyclopedia of Industrial Chemistry", 1 January 2005 (2005-01-01), Wiley-VCH Verlag, Weinheim, DE pages 1-28,

## Beschreibung

### HINTERGRUND DER ERFINDUNG

### 1. TECHNISCHES GEBIET

Die Erfindung betrifft ein Verfahren zur Durchführung einer In-Situ-Quench (ISQ) Reaktion durch Erzeugung eines reaktiven Zwischenproduktes (ZP) in Gegenwart eines geeigneten Reaktionspartners mit Hilfe eines Mikroreaktors.

### 2. STAND DER TECHNIK

Das ISQ-Konzept eröffnet vielfältige neue Chancen für die Organische Synthese, insbesondere im Zusammenhang mit Reaktionen, bei denen eine Organo-LithiumVerbindung durch (schnellen) Halogen-Lithium-Austausch in Gegenwart eines Elektrophils erzeugt und unmittelbar (in situ bzw. im Entstehen) abgefangen wird. Beispiele hierfür gab es bislang nur in einigen intramolekularen Reaktionen und (kürzlich) in der Herstellung von Arylboronsäuren bzw. deren Estern.

Ein altes, selbstverständliches Konzept der Chemie besteht darin reaktive Zwischenstufen in Gegenwart von geeigneten Reaktionspartnern zu erzeugen, und so direkt abzufangen. Neusprachlich kann man eine solche Vorgehensweise als "in-situ quench" (ISQ) bezeichnen. In der Synthese wird die ISQ-Technik z.B. häufig gezielt genutzt um reaktive Komponenten für Cycloadditionen in-situ zu generieren und direkt abzufangen. Bekannte Beispiele hierfür sind Dehydrobenzole oder o-Chinodimethane in Diels-Alder-Reaktionen (siehe z.B.: J. L. Segura, N. Martin, Chem. Rev. 1999, 99, 3199-3246). Völlig selbstverständlich ist diese Technik in radikalischen und carbokationischen Transformationen, bei denen kurzlebige Intermediate fast ausnahmslos in Gegenwart der Reaktionspartner generiert werden.

Die ISQ-Technik steht in einem Gegensatz zur schrittweisen Reaktionsführung, bei der zunächst ein aktives Reagenz separat erzeugt und dann erst in einer zweiten Syntheseoperation mit einem Reaktionspartner umgesetzt wird. Gerade in der Chemie von reaktiven Carbanionen (Enolat-Anionen, Grignard-Verbindungen, Organolithium-Verbindungen) ist es üblich, die reaktiven Zwischenstufen zunächst zu erzeugen (meist durch Deprotonierung oder Metal-Halogen-Austausch) um sie dann anschließend gezielt (und oft bei tiefen Temperaturen) mit Elektrophilen umzusetzen. In der Carbanionenchemie sind derartige Zweistufen-Verfahren "normal", während die ISQ-Methodik die seltene Ausnahme bildet.

1983 erschien eine zunächst wenig beachtete Arbeit (T. D.Krizan und J. C. Martin, J. Am. Chem. Soc. 1983, 105, 6155-6157), in der die Möglichkeit aufgezeigt wurde, die aus der ortho-Lithiierung von Aromaten mit Lithiumtetramethylpiperidid (LiTMP) hervorgehenden Lithioaromaten mit einem von Anfang an präsenten Elektrophil (TMSC1) abzufangen. In einer konzeptionell analogen Arbeit stellte wenig später E.J. Corey sein "in situ quench protocol" vor (E. J. Corey, A. W. Gross, Tetrahedron Lett. 1984, 25, 495-498.). Auch er konnte zeigen, dass die zur Deprotonierung von Carbonylverbindungen gängigen sperrigen Amidbasen wie LDA bei tiefen Temperaturen mit TMSC1 nicht reagieren. Wenn er die Base zu einer Mischung der Carbonylverbindung und TMSC1 gab (ISQ-Methoded) anstatt das Enolatanion vorab zu erzeugen und dann mit TMSC1 zu quenchen, konnte er hohe Ausbeuten und Regioselektivitäten bei der Herstellung von Silylenolethern erzielen. Diese Methodik wurde rasch auch von anderen aufgegriffen und findet auch heute noch vielfältige Anwendung, u.a. auch bei der enantioselektiven Deprotonierung/Silylierung von prochiralen Substraten (Simpkins, Schmalz).

(Ausgewählte neuere Anwendungen der Corey-Methode: C. D. Graf, C. Malan, K. Harms, P. Knochel, J. Org. Chem. 1999, 64, 5581-5588; H. Suzuki, N. Yamazaki, C. Kibayashi, J. Org. Chem. 2001, 66, 1494-1496)

Die Addition von Organocupraten an (α,β-ungesättigte) Carbonylverbindungen in Gegenwart von Trimethylchlorsilan (TMSC1) und gegebenenfalls HMPA kann ebenfalls als ISQ-Prozess erachtet werden, wobei allerdings die Natur der abzufangenden Zwischenstufe nicht völlig klar scheint (Modern Organocopper Chemistry, Ed. N. Krause, Wiley-VCH, 2002, p. 333ff).

Eine interessante ISQ-Reaktion nutzte Overman in der Total Synthese von (+)-Aloperine über eine intramolekulare Diels-Alder Reaktion (A. D. Brosius, L. E. Overman, L. Schwink, J. Am. Chem. Soc. 1999, 121, 700-709). Hier wurde eine Mischung eines Methylsulfonamides und Diethylchlorphosphat mit Lithiumhexamethyldisilazid (LHMDS) versetzt, wobei das primäre aus der Deprotonierung des Methylsulfonamides hervorgegangene Anion in situ phosphoryliert und das Produkt direkt weiter zu dem Wittig-Horner-Reagenz deprotoniert wird. Anschließende Wittig-Reaktion und intramolekulare [4+2]-Zyklisierung ergeben das vierfach annelierte Grundgerüst des Naturstoffs.

Die Synthese von ortho-substituierten Arylboronsäureestern durch ISQ durch Deprotonierung erzeugter (instabiler) Lithio-Intermediate mit Trialkylboraten wurde von zwei Gruppen beschrieben (J. Kristensen, M. Lysen, P. Vedso, M. Begtrup, Org. Lett. 2001, 3, 1435 und S. Caron, J. M. Hawkins, J. Org. Chem. 1998, 63, 2054).

Weiterhin wurden die Lithiierung aryl- oder heteroaryl-substituierter Allylchloride unter ISQ-Bedingungen in Gegenwart von Carbonylverbindungen beschrieben. Es resultieren Epoxide. (S. Florio, L. Troisi, Tetrahedron Lett. 1996, 37, 4777-4780.)

Die Synthese von Arylboronsäuren aus den lithiierten Halogenaromaten gelang ebenfalls unter ISQ-Bedingungen (Li, W.; Nelson, D. P.; Jensen, M. S.; Hoerrner, R. S.; Cai, D.; Larsen, R. D.; Reider, P. J.; J. Org. Chem. 2002; 67, 5394). Im ersten Schritt wird 3-Brompyridin einer Halogen-Metall-Austausch-Reaktion unterworfen, wobei der ebenfalls in der Reaktionslösung vorhandene Triisopropylboronester unangetastet bleibt. Das 3-Lithiumpyridin reagiert anschließend zum Arylboronester, der mit HCl zur Boronsäure hydrolysiert wird.

Weiterhin sind Synthesen bekannt, bei denen die aus einem Halogen-Lithium-Austausch (oder Zinn-Lithium-Austausch) hervorgehenden Zwischenstufen direkt intramolekular abgefangen werden (z.B. E. Piers et al Org. Lett. 2001, vol 3(21), 3245-3247).

Beispielhaft für die Durchführung von Reaktionen im Mikroreaktor seien die folgenden Patentanmeldungen genannt: EP 1 160 241 "Verfahren zur Herstellung von Chinolon-3-carbonsäuren, WO 02/068403 "Kontinuierliches Verfahren zur Herstellung von Dihydropyronen", sowie die Beschreibungen zahlreicher weiterer Reaktionsbeispiele auf der Hompage der Firma CPC-Cellular Process Chemistry Systems GmbH (http://www.cpc-net.com/reactions.shtml).

Im Bereich der Organischen Synthesechemie haben Reaktionen von hochreaktiven metallorganischen Reagenzien (Grignard- oder Organolithium-Verbindungen etc.) in den letzten Jahrzehnten eine enorme Bedeutung erlangt. Aus verfahrenstechnischen Gründen bestehen immer noch Vorbehalte, diese leistungsfähige Chemie zu nutzen. Vor dem Hintergrund der Verfügbarkeit von Butyllithium und verwandten Reagenzien im Tonnen-Maßstab, ist es eine Herausforderung sichere und leistungsfähige Verfahren für die synthetische Nutzung zu entwickeln.

Während Reaktionen zwischen Arylbromiden und Butyllithium im Normalfall schrittweise (im Batchbetrieb) durchgeführt werden, liegt der vorliegenden Erfindung die Aufgabe zu Grunde, bei diesen Reaktionen die ISQ-Methodik einzusetzen. Es wurde nun überraschenderweise gefunden, dass die ISQ-Methodik vorteilhaft eingesetzt werden kann, wenn man solche Reaktionen in einem Mikroreaktor durchführt.

### KURZE ZUSAMMENFASSUNG DER ERFINDUNG

Gegenstand der Erfindung ist somit ein Verfahren zur Durchführung einer In-Situ-Quench (ISQ) Reaktion durch Erzeugung eines reaktiven Zwischenproduktes (ZP) in Gegenwart eines geeigneten Reaktionspartners, dadurch gekennzeichnet, dass man ein Gemisch bestehend aus einem Vorprodukt (VP), welche eine durch eine starke Base deprotonierbare organische Verbindung oder eine organische Halogenverbindung, die durch Metall-Halogen Austausch in eine metallorganische Verbindung überführt werden kann, ist, einem Elektrophil (RP) und gegebenenfalls einem inerten Verdünnungsmittel in einen Mikroreaktor einleitet und dort mit einem hochreaktiven Reagenz (RG), welches eine lithiumorganische Verbindung ist, die geeignet ist das VP in das reaktive ZP überzuführen, mischt und man aus dem erhaltenen Reaktionsgemisch, gegebenenfalls nach Durchlaufen einer Verweilzeit-Einheit, das durch Reaktion des ZP mit dem RP erhaltene Endprodukt isoliert.

### KURZE BESCHREIBUNG DER ZEICHNUNGEN

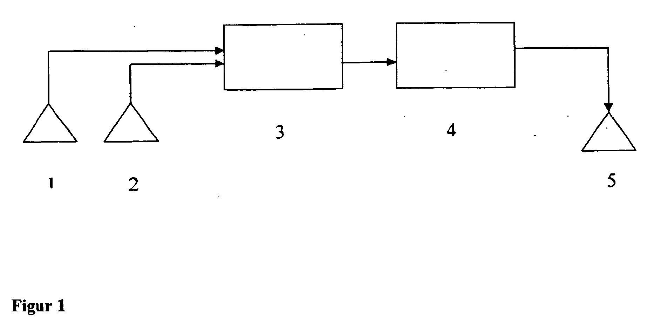

Figur 1 beschreibt den schematischen Aufbau eines Mikroreaktorsystems zur Durchführung einer ISQ Reaktion.

### DETAILLIERTE BESCHREIBUNG DER ERFINDUNG

Dabei können folgende Vorteile erzielt werden:
- einstufiges Verfahren;
- Zeitersparnis bei der Vorbereitung, Überwachung und Aufarbeitung;
- perfekte Temperaturkontrolle im Mikroreaktor (höhere Reaktionstemperaturen erlauben geringere Kosten für Kühlung der Anlagen);
- erhöhte Betriebssicherheit durch kleine Reaktionsvolumina und Stahlreaktoren;
- kontinuierliche Prozessführung mit der Möglichkeit der Produktion an 24 h / Tag;
- einfache Maßstabsvergrößerung durch Parallelisierung mehrer Reaktoren (numbering-up). Da die Bedingungen in jeder einzelnen Zelle gleich sind, ist kein Einfluss auf die Reaktionsparameter zu erwarten, wie beim klassischen scale-up Verfahren.

Vorzugsweise ist das RG eine lithiumorganische Verbindung, insbesondere ausgewählt aus der Gruppe bestehend aus *n*-Butyllithium, *sec*-Butyllithium, *tert-* Butyllithium, *n-*Hexyllithium, Methyllithium, Phenyllithium, Lithiumdiisopropylamid, Lithiumtetramethylpiperidid und Lithiumhexamethylsilazid ist..

Vorzugsweise ist das RP ein achirales, prochires oder chirales Elektrophil ausgewählt aus der Gruppe der gegebenenfalls substituierten Halogenalkane, wie zum Beispiel Methyljodid, Ethyljodid, n-Propylbromid, Pentylbromid oder Benzylbromid, Halogenalkene wie zum Beispiel Allylbromid, Oxirane, Aldehyde wie zum Beispiel Formaldehyd, Acetaldehyd, Propionaldehyd, Butyraldehyd oder Benzaldehyd, Ketone, wie zum Beispiel Aceton, Butanon, Cyclohexanon, Acetophenon oder Benzophenon, Carbonsäurederivate, wie zum Beispiel Carbonsäureester, Nitrile oder Anhydride, Trihydrocarbylsilylhalogenid, wie zum Beispiel Trimethylchlorsilan, tert-Butyldimethylchlorsilan oder tert-Butyl-diphenylchlorsilan, Acrylsäureester, wie zum Beispiel Accrylsäuremethylester, Methacrylsäuremethyester oder Acrylsäureethylester, α,β-ungesättigten Carbonylverbindungen, wie zum Beispiel Cyclohex-2-enon, 2-Bromacrylsäuremethylester Ethyl-vinylketon, Boronsäureester, wie zum Beispiel Trimethylborat, Phosphonsäureester, Alkylsulfonsäureester und Dialkylsulfate, wie zum Beispiel Dimethylsulfat oder Diethylsulfat.

Ganz besonders bevorzugt ist das RP ein gegebenenfalls substituertes, nicht-enolisierbares Keton mit einer pro-chiralen Carbonylgruppe, insbesondere Fenchon.

Besonders bevorzugt ist die Umsetzung von Arylbromiden mit Butyllithium zu Aryllithium-Verbindungen (Br-Li-Austausch) mit nachfolgender Umsetzung mit einem Elektrophil.

In einer bevorzugten Ausfiihrungsform wird das Gemisch aus dem VP und dem RP in einem Mikroreaktor mit dem RG gemischt, wobei die Mischungszone durch ein oder mehrere Wärmeelemente auf einer konstanten Temperatur von -40 ° bis + 50 °C, insbesondere von -20 ° bis + 30 °C gehalten wird.

In einer weiteren bevorzugten Ausführungsform wird das Gemisch aus dem VP und dem RP in einem Mikroreaktor mit dem RG bei einer Flussrate von 0,5 bis 20 ml/min, insbesondere 0,8 bis 10 ml/min, gemischt.

Als Mikroreaktoren kommen zum Beispiel solche, die in den europäischen Patentanmeldungen EP 0 688 242, EP 1 031 375, EP 1 123 734, EP 1 123 735, EP 1 125 630 oder dem US Patent US 5,811,062 beschrieben werden, in Betracht.

Zwischen einem Gehäusedeckel und einem Gehäuse sind eine Anzahl von Funktionsmodulen angeordnet, die durch den Zusammenbau unter Druck gehalten werden, um Abdichtflächen zwischen den Modulen zusammenzupressen. Jedes Funktionsmodul enthält eine Modulhälfte, die jeweils rahmenartig gestaltet sind, um Abdichtflächen darzubieten, die beim Aufeinanderpressen der Hälften abdichten. Im Deckel sind Fluidanschlüsse angebracht, die sich in Fluidkanälen durch die Randbereiche der Funktionsmodule fortsetzen. Von dort gibt es horizontale Kanäle zu Funktionsräumen, die in der Regel ein Kanalsystem oder Labyrinthsystem beinhalten. Die Kanäle der Funktionsräume aufen im allgemeinen schräg oder quer zueinander.

Die aus zwei Hälften zusammengebauten Funktionsmodule zeigen an ihren Oberflächen jeweils Öffnungen der Kanäle, die in standardisierten Abständen angeordnet sind, um beim Stapelaufbau von Funktionsmodulen genau zueinander zu fluchten. Diese Öffnungen sind mit Dichtstrukturen versehen, um fortlaufende Kanäle abdichtend zu kuppeln.

Im dargestellten Ausführungsbeispiel sei angenommen, dass ein Funktionsmodul einen Wärmetauscher darstellt, der aus einer ersten Wärmetauscherhälfte für Kühl- und/oder Heizmedium und einer zweiten Wärmetauscherhälfte für Reaktantenführung besteht. Das folgende Funktionsmodul ist ein Mischer für zwei Reaktionspartner. Das darauf folgende Funktionsmodul stellt eine Verweilstrecke dar, die aus einer Verweilstreckenhälfte für Reaktionsprodukt und einer Verweilstreckenhälfte für Kühl- und/oder Heizmedium besteht.

Vorzugsweise weisen die Mikrostrukturen im Bereich der Reaktanten- und der Reaktionsgemisch-Führung Kanalbreiten von 50-1000 µm, insbesondere 100-700 µm und Kanalhöhen von 50-800 µm, insbesondere 100-600 µm auf, und die Mikrostrukturen weisen im Bereich der Wärmeträger-Führung Kanalbreiten von 20-800 µm, insbesondere 100-600 µm und Kanalhöhen von 50-400 µm, insbesondere 100-300 µm auf, wobei vorzugsweise Geometrien verwendet werden, die keine Totwasserzonen ausbilden.

Da solche Mikroreaktoren gewöhnlich kontinuierlich betrieben werden, ist es nicht empfehlenswert, das aus dem Mikroreaktor kommende Reaktionsgemisch in einen herkömmlichen Reaktor zu füllen und dort verweilen zu lassen.

Optimale Verweilzeitmodule sind zum Beispiel in de europäischen Patentanmeldungen EP 1 157 738 und EP 1 188 476 beschrieben.

Solche Verweilzeitmodule dienen zur thermischen Nachbehandlung chemischer Reaktionsmedien aus Mikroreaktoren.

Der Begriff Verweilzeit bedeutet vorstehend und nachfolgend die Zeit, die dem Reaktionsmedium zur Nachreaktion nach der Durchmischung in dem erfindungsgemäßen Modul zur Verfügung gestellt wird. In der Regel ist sie abhängig von der Durchflussgeschwindigkeit und dem Volumen der zwei- oder dreidimensional gewundenen, bevorzugt spiral- oder schraubenförmigen Vertiefung auf mindestens einer Seite des Formkörpers. Das Volumen der Vertiefung lässt sich durch die Länge und den Durchmesser des Moduls, die Ganghöhe der schraubenförmigen Windung sowie die geometrische Lage der Produktentnahmeöffnung vorbestimmen.

Bevorzugt ist ein Verfahren zur Herstellung einer Verbindung der Formel (I), 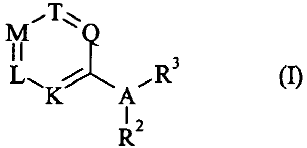 worin
- K, L, N, T und Q,: jeweils unabhängig voneinander für CR¹ oder N stehen;
- A für B,: CR⁴ oder SiR⁵ steht
- R¹: jeweils unabhängig voneinander Wasserstoff, C₁₋₆ Alkyl, C₁₋₆ Alkoxy, C₃₋₈ Cycloalkyl, Aryl, Aryloxy, Aryl-C₁₋₆-alkyl, Aryl-C₁₋₆-alkoxy oder Fluor bedeuten;
- R² und R³: im Falle A = CR⁴, jeweils unabhängig voneinander Wasserstoff, C₁₋₆ Alkyl, C₁₋₆Alkoxy, C₃₋₈ Cycloalkyl, Aryl, Aryloxy, Aryl-C₁₋₆-alkyl oder Aryl-C₁₋₆-alkoxy bedeuten;
- R² und R³: im Falle A = CR⁴, zusammengenommen mit dem eingeschlossenen CR⁴ einen mono- oder bicyclischen Ring mit 4 bis 8 Kohlenstoffatomen bilden;
- R² und R³: im Falle A = SiR⁵, jeweils unabhängig voneinander C₁₋₆ Alkyl, Aryl oder Aryl-C₁₋₆-alkyl bedeuten;
- R² und R³: im Falle A = B, jeweils unabhängig voneinander Hydroxy, C₁₋₆ Alkoxy, Aryloxy oder Aryl-C₁₋₆-alkyloxy bedeuten;
- R² und R³ O-: im Falle A = B, zusammengenommen eine Gruppe der Formel -O-(CH₂)ₘ-bilden;
- R⁴: Wasserstoff oder Hydroxy bedeutet;
- R⁵: C₁₋₆ Alkyl, Aryl oder Aryl-C₁₋₆-alkyl bedeutet; wobei man ein Gemisch bestehend aus einer Verbindung der Formel (II), 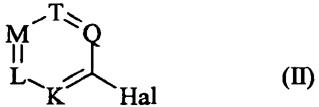
worin
K, L, M, T und Q die für Formel (I) angegebene Bedeutung aufweisen; und
Hal für Chlor, Brom oder Jod steht;
und einer Verbindung der Formel (III), 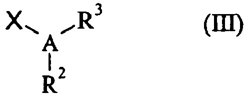 worin
- R² und R³: jeweils die für Formel (I) angegebene Bedeutung aufweisen, und A-X für B-OR⁶, Si-Y, CH-Y oder C=O steht,
- R⁶: C₁₋₆ Alkoxy, Aryloxy oder Aryl-C₁₋₆-alkyloxy bedeutet; und
- Y: für eine geeignete Abgangsgruppe steht,
in einem Mikroreaktor mit einer lithiumorganischen Verbindung mischt.

Besonders bevorzugt ist ein Verfahren zur Herstellung einer Verbindung der Formel (I), worin
einer der Gruppen K, L, N, T und Q, jeweils unabhängig voneinander für CR¹ oder N steht und die anderen CR¹ bedeuten;
- A für B,: CR⁴ oder SiR⁵ steht
- R¹: jeweils unabhängig voneinander Wasserstoff, C₁₋₃ Alkyl, C₁₋₃ Alkoxy, C₃₋₆ Cycloalkyl, Phenyl, Phenoxy, Benzyl, Benzyloxy oder Fluor bedeuten;
- R² und R³: im Falle A = CR⁴, jeweils unabhängig voneinander Wasserstoff, C₁₋₃ Alkyl, C₃₋₈ Cycloalkyl, Phenyl oder Benzyl bedeuten;
- R² und R³: im Falle A = CR⁴, zusammengenommen mit dem eingeschlossenen CR⁴ einen mono- oder bicyclischen Ring mit 5 bis 8 Kohlenstoffatomen bilden;
- R² und R³: im Falle A = SiR⁵, jeweils unabhängig voneinander C₁₋₄ Alkyl, Phenyl oder Benzyl bedeuten;
- R² und R³: im Falle A = B, jeweils unabhängig voneinander Hydroxy, C₁₋₃ Alkoxy, Phenoxy oder Benzyloxy bedeuten;
- R² und R³: im Falle A = B, zusammengenommen eine Gruppe der Formel -O-(CH₂)₂-O- bilden;
- R⁴: Wasserstoff oder Hydroxy bedeutet;
- R⁵: C₁₋₃ Alkyl, Phenyl oder Benzyl bedeutet.

Das erfindungsgemäße Verfahren erlaubt es ISQ Reaktionen in guten Ausbeute und Reinheit, insbesondere mit einem hohen Enantiomeren- bzw. Diastereomerenüberschuss bei Einsatz chiraler Ausgangsmaterialien, herzustellen. Durch Parallelisierung, größere Reaktionsvolumina, Arbeiten bei höheren Temperaturen oder unter erhöhtem Druck können die Durchsatzraten noch weiter gesteigert werden. Der Prozess lässt sich weitgehend automatisieren und ist kontinuierlich durchführbar. Daher ist der erfindungsgemäße Prozess wesentlich wirtschaftlicher durchzuführen als die arbeitsintensiven herkömmlichen Batch-Verfahren.

Um das Verständnis der vorliegenden Erfindung zu erleichtern werden die nachfolgenden illustrativen Beispiele für mögliche Reaktionstypen dargelegt. Die vorliegende Erfindung ist nicht beschränkt auf diese spezifischen Ausführungsformen, sondern umfasst den vollen Umfang der Patentansprüche.

### Beispiele

### Beispiele 1 bis 7

Umsetzung von 2-Brompyridin mit Fenchon durch Halogen-Metall-Austausch mit n-Butyllithium.
Ein Gemisch aus 10 mmol 2-Brompyridin und 20 mmol Fenchon wird in Mikroreaktor mit 15 mmol n-Butyllithium in Tetrahydrofuran gemischt. Die jeweiligen Reaktionsbedingungen können der nachfolgenden Tabelle I entnommen werden. Dabei erzielt man die in der Tabelle I aufgeführten Ergebnisse:

**Tabelle I**

| | | | | | |
|---|---|---|---|---|---|
| 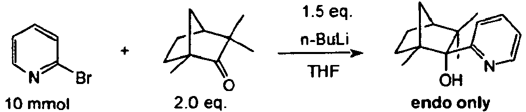 | | | | | |

| Beispiel-Nr. | Temperatur [°C] | Flußrate [ml/min] | Residenz-Zeit [min] | Isolierte Ausbeute [%] | d.e. [%]* |
|---|---|---|---|---|---|
| 1 | -25 | 5 | 3 | 71 | 100 |
| 2 | 0 | 5 | 3 | 64 | 100 |
| 3 | -25 | 3 | 5 | 65 | 100 |
| 4 | -25 | 1 | 15 | 65 | 100 |
| 5** | -25 | 5 | 3 | 43 | 100 |
| 6*** | -25 | 5 | 3 | 46 | 100 |
| 7*** | -25 | 1 | 15 | 57 | 100 |

| | | | | | |
|---|---|---|---|---|---|
| *) Gaschromatographisch bestimmt **) 2-Brompyridin : Fenchon : n-BuLi = 1 : 2 : 1 ***) 2-Brompyridin : Fenchon : n-BuLi = 1 : 1 : 1 | | | | | |

Die Reaktion wurde in einem Mikroreaktorsystem gemäß Fig. 1 durchgeführt, wobei die Einheit (1) beispielhaft die Zufuhr des Gemisches von 2-Brompyridine und Fenchon darstellt und die Einheit (2) die Zufuhr von n-Butyllithium darstellt. Die Mikroreaktoreinheit (3) weist beispielhaft ein Volumen von 2 ml auf und die Residenzzeiteinheit (4) ein Volumen von 30 ml. In der Einheit (5) wird das hergestellt Produkt aufgefangen.

### Zum Vergleich:

Die Ergebnisse der schrittweisen Durchführung derselben Reaktion in einem Reaktionskolben werden in Tabelle II gezeigt:

**Tabelle II**

| | | | |
|---|---|---|---|
| 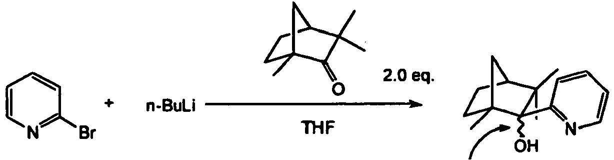 | | | |

| Vergleichsbeispiel-Nr. | Temperatur [°C] | Isolierte Ausbeute [%] | d.e. [% ]* |
|---|---|---|---|
| 1 | -70 | 88 | 100 (endo) |
| 2 | -30 | 63 | 51 (endo) |
| 3 | 0 | <7 | 9 (exo) |
| 4** | -78 | 56 | 100 (endo) |

| | | | |
|---|---|---|---|
| *) Gaschromatographisch bestimmt **) J. Organomet. Chem. 603 (2000), 69-79 2-Brompyridin : Fenchon : n-BuLi = 1 : 1,05 : 1,05; Lösungsmittel Diethylether | | | |

Dass unter den Bedingungen bei höheren Temperaturen eine hohe Diasteroselektivität erzielt wurde, kann so interpretiert werden, dass das frisch (in situ) entstehende Lithiopyridin sich chemisch von der (nur formal identischen) Spezies unterscheidet, die im zweistufigen Verfahren separat hergestellt wird, in viel höherer Konzentration vorliegt und Zeit hat, die thermodynamisch stabileren Aggregate (Cluster) auszubilden.

### Beispiele 8 bis 11

Analog Beispiel 1 werden folgende Reaktionen gemäß dem allgemeinen Schema: 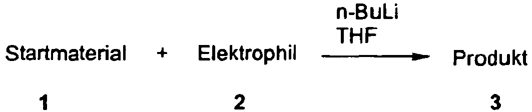 durchgeführt.

Die Ergebnisse dieser Reaktionen fasst die folgende Tabelle II zusammen.

**Tabelle II**

| Nr. | 1 | 2 | 3 | Ausbeute (Kolben, T=0°C) | Ausbeute (MR, T=25°C) |
|---|---|---|---|---|---|
| 8 | 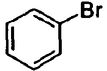 | Me₂SO₄ | 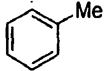 | 33 | 58 |
| 9 | 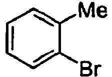 | TMSCl | 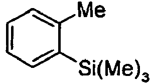 | 88 | 77 |
| 10 | 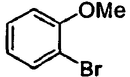 | TMSCl | 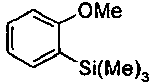 | 91 | 87 |
| 11 | 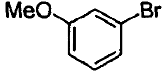 | Me₂SO₄ | 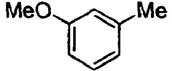 | 44 | 61 |

Die angeführten Ergebnisse zeigen vergleichbare bis bessere Ausbeuten bei der ISQ-Reaktion im Mikroreaktor im Vergleich mit der Reaktionsführung im Kolben.

### Beispiel 12

Folgende Reaktion wurde im Vergleich ISQ-Reaktion im Mikroreaktor und konventionell (Kolben) durchgeführt:

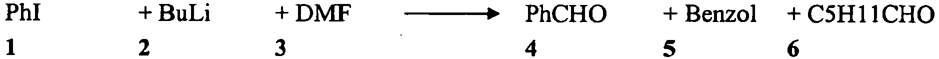

### ISQ-Reaktion konventionell (Kolben)

| **Temp.** | **% 4** | **% 5** | **% 6** |
|---|---|---|---|
| - 35 °C | 40 | 9 | 2,5 |

### ISQ-Reaktion im Mikroreaktor

| **Temp.** | **% 4** | **% 5** | **% 6** |
|---|---|---|---|
| 0 °C | 39 | 8 | 2 |

## Patentansprüche

1. Verfahren zur Durchführung einer In-Situ-Quench (ISQ) Reaktion durch Erzeugung eines reaktiven Zwischenproduktes (ZP) in Gegenwart eines geeigneten Reaktionspartners, **dadurch gekennzeichnet, dass** man ein Gemisch bestehend aus einem Vorprodukt (VP), welche eine durch eine starke Base deprotonierbare organische Verbindung oder eine organische Halogenverbindung, die durch Metall-Halogen Austausch in eine metallorganische Verbindung überführt werden kann, ist, einem Elektrophil (RP) und gegebenenfalls einem inerten Verdünnungsmittel in einen Mikroreaktor einleitet und dort mit einem hochreaktiven Reagenz (RG), welches eine lithiumorganische Verbindung ist, die geeignet ist das VP in das reaktive ZP überzuführen, mischt und man aus dem erhaltenen Reaktionsgemisch, gegebenenfalls nach Durchlaufen einer Verweilzeit-Einheit, das durch Reaktion des ZP mit dem RP erhaltene Endprodukt isoliert.

2. Verfahren nach einem der Anspruch 1, **dadurch gekennzeichnet, dass** das RG eine lithiumorganische Verbindung vorzugsweise ausgewählt aus der Gruppe bestehend aus *n*-Butyllithium, *sec*-Butyllithium, *tert-* Butyllithium, *n*-Hexyllithium, Methyllithium, Phenyllithium, Lithiumdiisopropylamid, Lithiumtetramethylpiperidid und Lithiumhexamethylsilazid ist.

3. Verfahren nach einem der Ansprüche 1 oder 2, **dadurch gekennzeichnet, dass** das RP ein achirales, prochires oder chirales Elektrophil ausgewählt aus der Gruppe der gegebenenfalls substituierten Halogenalkane, Halogenalkene, Oxirane, Aldehyde, Ketone, Carbonsäurederivate, Trihydrocarbylsilylhalogenid, Acrylsäureester, α,β-ungesättigten Carbonylverbindungen, Boronsäureester, Phosphonsäureester, Alkylsulfonsäureester und Dialkylsulfate ist.

4. Verfahren nach einem der Ansprüche 1 bis 3, **dadurch gekennzeichnet, dass** das RP ein gegebenenfalls substituertes, nicht-enolisierbares Keton mit einer pro-chiralen Carbonylgruppe ist.

5. Verfahren nach einem der Ansprüche 1 bis 4, **dadurch gekennzeichnet, dass** das Gemisch aus dem VP und dem RP in einem Mikroreaktor mit dem RG gemischt wird, wobei die Mischungszone durch ein oder mehrere Wärmeelemente auf einer konstanten Temperatur von -40 ° bis + 50 °C gehalten wird.

6. Verfahren nach einem der Ansprüche 1 bis 5, **dadurch gekennzeichnet, dass** das Gemisch aus dem VP und dem RP in einem Mikroreaktor mit dem RG bei einer Flussrate von 0,5 bis 20 ml/min gemischt wird.

7. Verfahren nach einem der Ansprüche 1 bis 6 zur Herstellung einer Verbindung der Formel (I), 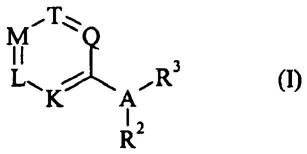 worin
K, L, N, T und Q, jeweils unabhängig voneinander für CR¹ oder N stehen;
A für B, CR⁴ oder SiR⁵ steht
R¹ jeweils unabhängig voneinander Wasserstoff, C₁₋₆ Alkyl, C₁₋₆ Alkoxy, C₃₋₈ Cycloalkyl, Aryl, Aryloxy, Aryl-C₁₋₆-alkyl, Aryl-C₁₋₆-alkoxy oder Fluor bedeuten;
R² und R³ im Falle A = CR⁴, jeweils unabhängig voneinander Wasserstoff, C₁₋₆ Alkyl, C₁₋₆ Alkoxy, C₃₋₈ Cycloalkyl, Aryl, Aryloxy, Aryl-C₁₋₆-alkyl oder Aryl-C₁₋₆-alkoxy bedeuten;
R² und R³ im Falle A = CR⁴, zusammengenommen mit dem eingeschlossenen CR⁴ einen mono- oder bicyclischen Ring mit 4 bis 8 Kohlenstoffatomen bilden;
R² und R³ im Falle A = SiR⁵, jeweils unabhängig voneinander C₁₋₆ Alkyl, Aryl oder Aryl-C₁₋₆-alkyl bedeuten;
R² und R³ im Falle A = B, jeweils unabhängig voneinander Hydroxy, C₁₋₆ Alkoxy, Aryloxy oder Aryl-C₁₋₆-alkyloxy bedeuten;
R² und R³ im Falle A = B, zusammengenommen eine Gruppe der Formel -O-(CH₂)ₘ-O-bilden ;
R⁴ Wasserstoff oder Hydroxy bedeutet;
R⁵ C₁₋₆ Alkyl, Aryl oder Aryl-C₁₋₆-alkyl bedeutet;
**dadurch gekennzeichnet, dass** man ein Gemisch bestehend aus einer Verbindung der Formel (II), 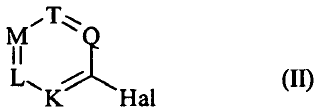 worin
K, L, M, T und Q die für Formel (I) angegebene Bedeutung aufweisen; und
Hal für Chlor, Brom oder Jod steht;
und einer Verbindung der Formel (III), 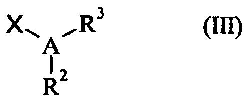 worin
R² und R³ jeweils die für Formel (I) angegebene Bedeutung aufweisen, und A-X für B-OR⁶, Si-Y, CH-Y oder C=O steht,
worin
R⁶ C₁₋₆ Alkoxy, Aryloxy oder Aryl-C₁₋₆-alkyloxy bedeutet; und
Y für eine geeignete Abgangsgruppe steht,
in einem Mikroreaktor mit einer lithiumorganischen Verbindung mischt.

8. Verwendung eines Mikroreaktors zur Durchführung einer In-Situ-Quench (ISQ) Reaktion, wobei ein Gemisch bestehend aus einem Vorprodukt (VP), welches eine durch eine starke Base deprotonierbare organische Verbindung oder eine organische Halogenverbindung, die durch Metall-Halogen Austausch in eine metallorganische Verbindung überführt werden kann, ist, einem Elektrophil (RP) und gegebenenfalls einem inerten Verdünnungsmittel in den Mikroreaktor eingeleitet wird und dort mit einem hochreaktiven Reagenz (RG), welches eine lithiumorganische Verbindung ist, die geeignet ist das VP in ein reaktives ZP überzuführen, gemischt wird.

## Claims

1. Process for performing an in-situ quench (ISQ) reaction by generating a reactive intermediate (I) in the presence of a suitable reaction partner, **characterized in that** a mixture consisting of a precursor (PC), which is an organic compound which can be deprotonated by a strong base, or is an organohalogen compound which can be converted to an organometallic compound by metal-halogen exchange, an electrophile (EP) and optionally an inert diluent is introduced into a microreactor and is mixed therein with a highly reactive reagent (RG), which is an organolithium compound suitable for converting the PC to the reactive I, and the end product obtained by reaction of the I with the EP is isolated from the resulting reaction mixture, optionally after passing through a residence time unit.

2. Process according to Claim 1, **characterized in that** the RG is an organolithium compound preferably selected from the group consisting of n-butyllithium, *sec*-butyllithium, *tert-*butyllithium, *n*-hexyllithium, methyllithium, phenyllithium, lithium diisopropylamide, lithium tetramethylpiperidide and lithium hexamethylsilazide.

3. Process according to either of Claims 1 and 2, **characterized in that** the EP is an achiral, prochiral or chiral electrophile selected from the group of the optionally substituted haloalkanes, haloalkenes, oxiranes, aldehydes, ketones, carboxylic acid derivatives, trihydrocarbylsilyl halide, acrylic acid esters, α,β-unsaturated carbonyl compounds, boronic acid esters, phosphonic acid esters, alkylsulphonic acid esters and dialkyl sulphates.

4. Process according to any of Claims 1 to 3, **characterized in that** the EP is an optionally substituted non-enolizable ketone having a prochiral carbonyl group.

5. Process according to any of Claims 1 to 4, **characterized in that** the mixture of the PC and the EP is mixed with the RG in a microreactor, in which the mixing zone is kept at a constant temperature of -40° to +50°C by one or more heating elements.

6. Process according to any of Claims 1 to 5, **characterized in that** the mixture of the PC and the EP is mixed with the RG at a flow rate of 0.5 to 20 mL/min in a microreactor.

7. Process according to any of Claims 1 to 6 for preparing a compound of the formula (I), 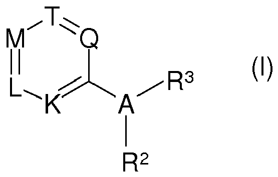 where
K, L, M, T and Q each independently are CR¹ or N;
A is B, CR⁴ or SiR⁵;
R¹ each independently are hydrogen, C₁₋₆ alkyl, C₁₋₆ alkoxy, C₃₋₈ cycloalkyl, aryl, aryloxy, aryl-C₁₋₆-alkyl, aryl-C₁₋₆-alkoxy or fluorine;
R² and R³ in the case that A = CR⁴, each independently are hydrogen, C₁₋₆ alkyl, C₁₋₆ alkoxy, C₃₋₈ cycloalkyl, aryl, aryloxy, aryl-C₁₋₆-alkyl or aryl-C₁₋₆-alkoxy;
R² and R³ in the case that A = CR⁴, form a mono- or bicyclic ring having 4 to 8 carbon atoms, which includes the CR⁴;
R² and R³ in the case that A = SiR⁵, each independently are C₁₋₆ alkyl, aryl or aryl-C₁₋₆-alkyl;
R² and R³ in the case that A = B, each independently are hydroxy, C₁₋₆ alkoxy, aryloxy or aryl-C₁₋₆-alkyloxy;
R² and R³ in the case that A = B, taken together form a group of the formula -O- (CH2)ₘ-O-;
R⁴ is hydrogen or hydroxy;
R⁵ is C₁₋₆ alkyl, aryl or aryl-C₁₋₆-alkyl;
**characterized in that** a mixture consisting of a compound of the formula (II), 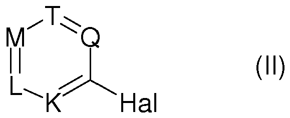 where
K, L, M, T and Q have the meaning stated for formula (I); and
Hal is chlorine, bromine or iodine;
and a compound of the formula (III), 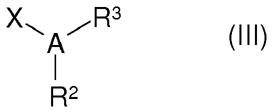 where
R² and R³ each have the meaning stated for formula (I), and
A-X is B-OR⁶, Si-Y, CH-Y or C=O,
where
R⁶ is C₁₋₆ alkoxy, aryloxy or aryl-C₁₋₆-alkyloxy; and
Y is a suitable leaving group,
are mixed with an organolithium compound in a microreactor.

8. Use of a microreactor for performing an in-situ quench (ISQ) reaction, where a mixture consisting of a precursor (PC), which is an organic compound which can be deprotonated by a strong base, or is an organohalogen compound which can be converted to an organometallic compound by metal-halogen exchange, an electrophile (EP) and optionally an inert diluent is introduced into the microreactor and is mixed therein with a highly reactive reagent (RG), which is an organolithium compound suitable for converting the PC to a reactive I.

## Revendications

1. Procédé pour la mise en oeuvre d'une réaction In-Situ-Quench (ISQ) par production d'un produit intermédiaire (ZP) réactif, en présence d'un partenaire réactionnel approprié, **caractérisé en ce qu'**on fait passer dans un microréacteur un mélange constitué d'un produit précurseur (VP), qui est un composé organique déprotonable par une base forte ou un composé organique halogéné, qui peut être converti par échange métal-halogène en un composé organométallique, d'un composé électrophile (RP) et éventuellement d'un diluant inerte et l'y mélange avec un réactif (RG) hautement réactif, qui est un composé organolithié qui est approprié à convertir le VP en le ZP réactif, et on isole à partir du mélange réactionnel obtenu, éventuellement après avoir traversé une unité pour temps de séjour, le produit final obtenu par la réaction du ZP avec le RP.

2. Procédé selon la revendication 1, **caractérisé en ce que** le RG est un composé organolithié de préférence choisi dans le groupe constitué par le n-butyllithium, le sec-butyllithium, le tert-butyllithium, le n-hexyllithium, le méthyllithium, le phényllithium, le diisopropylamidure de lithium, le tétraméthylpipéridide de lithium et l'hexaméthyl-silazide de lithium.

3. Procédé selon la revendication 1 ou 2, **caractérisé en ce que** le RP est un composé électrophile achiral, prochiral ou chiral, choisi dans le groupe constitué par des halogénoalcanes, halogénoalcènes, oxiranes, aldéhydes, cétones, dérivés d'acides carboxyliques, un halogénure de trihydrocarbylsilyle, des esters d'acide acrylique, composés carbonyle α,β-insaturés, esters d'acide boronique, esters d'acides phosphonique, esters d'acides alkylsulfoniques et sulfates de dialkyle, éventuellement substitués.

4. Procédé selon l'une quelconque des revendications 1 à 3, caractérisé en ce le RP est une cétone non énolisable, éventuellement substituée, comportant un groupe carbonyle prochiral.

5. Procédé selon l'une quelconque des revendications 1 à 4, **caractérisé en ce qu'**on mélange le mélange du VP et du RP dans un microréacteur avec le RG, en maintenant la zone de mélange à une température constante de -40° à +50 °C au moyen d'un ou plusieurs éléments de chauffage.

6. Procédé selon l'une quelconque des revendications 1 à 5, caractérisé en qu'on mélange le mélange du VP et du RP dans un microréacteur avec le RG, à un débit de 0,5 à 20 ml/min.

7. Procédé selon l'une quelconque des revendications 1 à 6, pour la préparation d'un composé de formule (I), 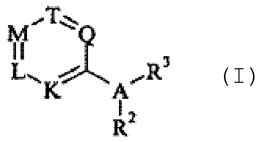 dans laquelle
K, L, M, T et Q représentent chacun, indépendamment les uns des autres, CR¹ ou N ;
A représente B, CR⁴ ou SiR⁵
R¹ représente chaque fois indépendamment un atome d'hydrogène, un groupe alkyle en C₁-C₆, alcoxy en C₁-C₆, cycloalkyle en C₃-C₈, aryle, aryloxy, aryl-alkyle(C₁-C₆), aryl-alcoxy(C₁-C₆) ou un atome de fluor ;
R² et R³ dans le cas où A = CR⁴, représentent chacun indépendamment l'un de l'autre un atome d'hydrogène, un groupe alkyle en C₁-C₆, alcoxy en C₁-C₆, cycloalkyle en C₃-C₈, aryle, aryloxy, aryl-alkyle(C₁-C₆) ou aryl-alcoxy (C₁-C₆) ;
R² et R³ dans le cas où A = CR⁴, forment ensemble, avec le groupe CR⁴ inclus, un cycle mono- ou bicyclique ayant de 4 à 8 atomes de carbone ;
R² et R³ dans le cas où A = SiR⁵ représentent chacun indépendamment l'un de l'autre un groupe alkyle en C₁-C₆, aryle ou aryl-alkyle(C₁-C₆) ;
R² et R³ dans le cas où A = B, représentent chacun indépendamment l'un de l'autre un groupe hydroxy, alcoxy en C₁-C₆, aryloxy ou aryl-alkyloxy(C₁-C₆) ;
R² et R³ dans le cas où A = B, forment ensemble un groupe de formule -O-(CH₂)ₘ-O- ;
R⁴ représente un atome d'hydrogène ou le groupe hydroxy ;
R⁵ représente un groupe alkyle en C₁-C₆, aryle ou aryl-alkyle(C₁-C₆) ;
**caractérisé en ce qu'**on mélange un mélange constitué d'un composé de formule (II), 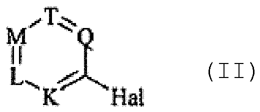 dans laquelle
K, L, M, T et Q ont la signification indiquée pour la formule (I) ; et
Hal représente un atome de chlore, brome ou iode ;
et un composé de formule (III) 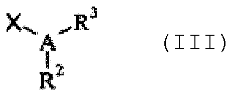 dans laquelle
R² et R³ ont chacun la signification indiquée pour la formule (I), et
A-X représente B-OR⁶, Si-Y, CH-Y ou C=O,
où
R⁶ représente un groupe alcoxy en C₁-C₆, aryloxy ou aryl-alkyloxy(C₁-C₆) ; et
Y représente un groupe partant convenable,
dans un microréacteur, avec un composé organolithié.

8. Utilisation d'un microréacteur pour la mise en oeuvre d'une réaction In-Situ-Quench (ISQ), dans laquelle on fait passer dans le microréacteur un mélange constitué d'un produit précurseur (VP), qui est un composé organique déprotonable par une base forte ou un composé organique halogéné, qui peut être converti par échange métal-halogène en un composé organométallique, d'un composé électrophile (RP) et éventuellement d'un diluant inerte et l'y mélange avec un réactif (RG) hautement réactif, qui est un composé organolithié qui est approprié à convertir le VP en un ZP réactif.
